# EUROPEAN PATENT SPECIFICATION

(11) **EP 2 768 417 B1**
(45) Date of publication and mention of the grant of the patent: **12.09.2018**
(21) Application number: 12780986.1
(22) Date of filing: 15.10.2012
(51) Int. Cl.: A61B 17/00

(54) **SYSTEM FOR SURGICAL TOOL TRACKING**
SYSTEM ZUR VERFOLGUNG EINES CHIRURGISCHEN WERKZEUGS
SYSTÈME DE SUIVI D'OUTIL CHIRURGICAL

(30) Priority: 18.10.2011 US 201113276048
(43) Date of publication of application: 27.08.2014
(73) Proprietor: Mako Surgical Corp., Fort Lauderdale, FL 33317 (US)
(72) Inventor: KANG, Hyosig, Weston, FL 33327 (US); NORTMAN, Scott, Sunrise, FL 33326 (US)
(74) Representative: Barker Brettell LLP
(86) International application number: PCT/US2012/060259
(87) International publication number: WO 2013/059125

(56) References cited:
- WO-A1-00/35366
- WO-A2-2008/118524
- US-A- 5 657 429
- US-A1- 2006 142 657
- US-A1- 2009 228 145
- US-B1- 6 228 089

## Description

### FIELD OF THE INVENTION

The present invention relates generally to surgical systems, and more specifically to systems for tracking positions and orientations of tools during surgical procedures.

### BACKGROUND

Minimally invasive surgery (MIS) is the performance of surgery through incisions that are considerably smaller than incisions used in traditional surgical approaches. For example, in an orthopedic application such as total knee replacement surgery, an MIS incision length may be in a range of about 10.2cm to 15.2 cm (4 to 6 inches), whereas an incision length in traditional total knee surgery is typically in a range of about 15.2cm to 30.5cm (6 to 12 inches). As a result of the smaller incision length, MIS procedures are generally less invasive than traditional surgical approaches, which minimizes trauma to soft tissue, reduces postoperative pain, promotes earlier mobilization, shortens hospital stays, and speeds rehabilitation.

MIS presents several challenges for a surgeon. For example, in minimally invasive orthopedic joint replacement, the small incision size may reduce the surgeon's ability to view and access the anatomy, which may increase the complexity of sculpting bone and assessing proper implant position. As a result, accurate placement of implants may be difficult. Conventional techniques for counteracting these problems include, for example, surgical navigation, positioning the subject patient limb for optimal joint exposure, and employing specially designed, downsized instrumentation and complex surgical techniques. Such techniques, however, typically require a large amount of specialized instrumentation, a lengthy training process, and a high degree of skill. Moreover, operative results for a single surgeon and among various surgeons are not sufficiently predictable, repeatable, and/or accurate. As a result, implant performance and longevity varies among patients.

To assist with MIS and conventional surgical techniques, advancements have been made to assist with understanding the spatial and rotational relationships between surgical instruments and tissue structures with which they are intervening during surgery. For example, various types of optical tracking configurations, such as those available from Northern Digital, Inc. of Ontario, Canada, have been utilized in surgery to track surgical instrument position. One of the challenges with optical tracking is that a line of sight generally must be maintained between markers on the tracked instrument and a sensing camera, and maintaining this line of sight, as well as a substantially optically debris-free marker state, may be suboptimal from a surgical operations perspective. There is a need for minimally invasive tracking technologies which are well suited for detecting positional and rotational information pertinent to surgical instruments relative to targeted tissue structures, with minimized surgical operation interference.

United States patent application US 2009/228145 A1 describes a method and apparatus for haptic hard surface emulation using a dynamic physical constraint. The movement and position of the dynamic physical constraint is actively controlled in order to emulate a hard surface. The dynamic physical constraint may be controlled by a computer. In another aspect, the dynamic physical constraint limits the motion of a manipulator joint in space. The position at any time of the dynamic physical constraint is dependent on the position in space of the manipulator's end effector.

International patent application WO2008118524 A2 describes an instrumented linkage system to facilitate accuracy and efficiency of a surgical procedure. The linkage system may be directly attached to a bone and used to register the bone to a computer. The linkage system may also be used to verify the accuracy and alignment of planned resections relative to the bone.

United States patent US 6228089 B1 describes a device for positioning and guiding a surgical instrument during orthopaedic interventions, comprising an industrial robot which has a program-controlled robot arm with several articulated joints and a mounting plate at the end of said arm, an instrument for the orthopaedic intervention attached to the mounting plate and a program control with computer. Sensors for detecting the position of the joints in an instrument-coordinating system defined in the program control are situated in the joints of the robot arm. Document US2006/142657 discloses an haptic guidance system including a surgical device, configured to be manipulated by a user to perform a procedure on a patient and a computer system.

### BRIEF DESCRIPTION OF THE DRAWINGS

Figure 1 illustrates some of the anatomy of a shoulder joint that may be subject to an orthopaedic intervention.
Figure 2 illustrates an orthopaedic surgery system comprising a bone removal instrument coupled to a patient's skeletal anatomy by a mechanical tracker.
Figure 3 illustrates a robotic surgery system comprising a base subsystem, a robotic instrument support structure, and an orthopaedic surgery instrument.
Figure 4A illustrates a mechanical tracker.
Figure 4B illustrates a partial cutaway view of a mechanical tracker.
Figure 4C illustrates a partial cutaway view of a mechanical tracker.
Figure 4D illustrates a partial cutaway view of a mechanical tracker.
Figure 4E illustrates a partial cutaway view of a mechanical tracker.
Figure 4F illustrates a close up orthogonal view of a kinematic interface member.
Figure 5 illustrates aspects of a surgical process using a mechanical tracker.
Figure 6 illustrates a partial cutaway view of a mechanical tracker that features potentiometers as joint rotation sensors.
Figure 7A illustrates one view of a calibration configuration.
Figure 7B illustrates another view of a calibration configuration.
Figure 8 illustrates a calibration process.

### SUMMARY

An aspect of the invention is provided as set out by claim 1 of the appended claims. Embodiments of the inventions are provided as set out by the appended dependent claims.

The surgical instrument may comprise a bone removal instrument. The surgical instrument may comprise a electromechanically-actuated burr. The surgical instrument may be coupled to an immobilized base unit by a linkage arm coupled to the at least one servo motor. The linkage arm may comprise a robotic arm, and the controller may be configured to selectively activate the at least one servo motor to enforce motion limitations upon the surgical instrument. The controller may be configured to provide haptic feedback to an operator handling the surgical instrument by controlled actuation of the one or more servo motors. The controller may be configured to provide corrective motion to the surgical instrument by controlled actuation of the one or more servo motors. The mechanical tracker linkage may comprise at least two substantially rigid portions coupled by at least one movable joint. The mechanical tracker linkage may comprise at least three substantially rigid portions coupled in a series configuration by two or more movable joints. The series configuration may comprise a proximal end and a distal end, each of which is coupled to a kinematic quick-connect fitting. A proximal kinematic quick-connect fitting may be configured to be fixedly and removably coupled to a skeletal bone. A distal kinematic quick-connect fitting may be configured to be fixedly and removably coupled to the surgical instrument. The proximal kinematic quick-connect fitting may be configured to be fixedly and removably coupled to the skeletal bone using an additional kinematic quick-connect fitting coupled to the skeletal bone. The distal kinematic quick-connect fitting may be configured to be fixedly and removably coupled to the surgical instrument using an additional kinematic quick-connect fitting coupled to the surgical instrument. The proximal and additional kinematic quick-connect fittings may be biased to stay in a coupled configuration by one or more magnets associated with one or more kinematic orienting surfaces. The distal and additional kinematic quick-connect fittings may be biased to stay in a coupled configuration by one or more magnets associated with one or more kinematic orienting surfaces. The additional kinematic quick-connect fitting may be coupled to one or more pins, which are fastened directly to the skeletal bone. At least one of the one or more joint rotation sensors may comprise an encoder. At least one of the one or more joint rotation sensors may comprise a potentiometer. The mechanical tracker linkage may comprise an on-board power supply configured to power the one or more joint rotation sensors. The tracker linkage may comprise a disposable polymeric material selected from the group consisting of: nylon, glass filled nylon, polyethylene terepthalate, polystyrene, polyethylene, and copolymers thereof.

A method is disclosed, of conducting robotic surgery on a bone of a patient, comprising coupling a proximal skeletal fastener to a skeletal structure near the bone; coupling a mechanical tracker linkage between the proximal skeletal fastener and a surgical instrument, the tracker linkage comprising one or more joints associated with one or more joint rotation sensors and being configured to send joint signals to a controller; and controlling positioning of the surgical instrument based at least in part upon the joint signals received from the mechanical tracker, and one or more servo motors operatively coupled to the controller. Coupling a proximal skeletal fastener may comprise fixedly coupling a pin to the skeletal structure near the bone. The bone of the patient may comprise a bone of the shoulder joint of the patient, and the skeletal structure near the bone may comprise a scapula of the patient. The bone of the patient may comprise a tibia of the patient, and the skeletal structure near the bone may comprise a femur of the patient. The method further may comprise removing a portion of the tissue comprising the bone of the patient, the surgical instrument comprising a bone-removal instrument. The bone-removal instrument may comprise a rotary burr, and removing a portion of the tissue comprising the bone may comprise controllably moving the burr. The method further may comprise transmitting the joint signals to the controller using a wired connection. The method further may comprise transmitting the joint signals to the controller using a wireless connection. The method further may comprise operating the controller to resist movements of the surgical instrument attempted by manipulation of the surgical instrument by an operator through actuation of the one or more servo motors coupled the movable instrument support structure. The method further may comprise operating the controller to provide corrective motion of the surgical instrument in response to attempted by manipulation of the surgical instrument by an operator through actuation of the one or more servo motors coupled the movable instrument support structure. The one or more servo motors may be operatively coupled to a movable instrument support structure configured to couple the surgical instrument to an immobilized mechanical base, and the movable instrument support structure may comprise a series of rigid linkages coupled by movable joints. The movable instrument support structure may be a robotic arm. Coupling the mechanical tracker linkage to the proximal skeletal fastener may comprise utilizing a removably couplable kinematic quick connect fitting. Coupling the mechanical tracker linkage to the surgical instrument may comprise utilizing a removably couplable kinematic quick connect fitting. Moving the surgical instrument may cause each of the mechanical tracker linkage and the movable instrument support structure to move without colliding with each other in a surgical range of motion when an end effector coupled to the surgical instrument is near a portion of the bone of the patient to be operated upon. The controller may be further operated to impart haptic feedback to the operator through selected actuation of the one or more servo motors. The method further may comprise intraoperatively decoupling the mechanical tracker linkage from the proximal skeletal fastener. The method further may comprise intraoperatively decoupling the mechanical tracker linkage from the surgical instrument. The method further may comprise registering the mechanical tracker linkage and instrument support structure movement relative to each other by moving the surgical instrument and receiving signals at the controller from both the mechanical tracker linkage and instrument support structure movement. The method further may comprise calibrating movement of the mechanical tracker linkage relative to movement of the instrument support structure by moving the surgical instrument and receiving signals at the controller from both the mechanical tracker linkage and instrument support structure movement. The method further may comprise switching an end effector coupled to the surgical instrument and recalibrating movement of the mechanical tracker linkage relative to movement of the instrument support structure by moving the surgical instrument and receiving signals at the controller from both the mechanical tracker linkage and instrument support structure movement. At least one of the one or more joint rotation sensors may comprise an encoder. At least one of the one or more joint rotation sensors may comprise a potentiometer. The method further may comprise calibrating the potentiometer using an encoder. The method further may comprise generating calibration information while calibrating, and storing said calibration information on a memory device operatively coupled to the potentiometer.

### DETAILED DESCRIPTION

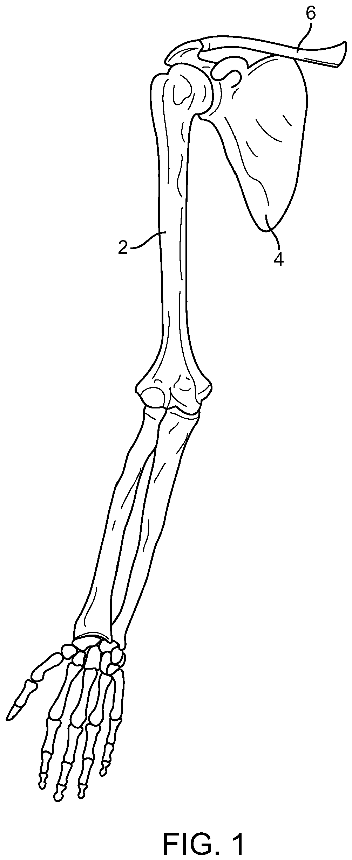

As described above, certain surgical techniques have evolved to rely upon a detailed understanding of the spatial and rotational positioning of surgical instruments relative to targeted tissues. For example, in certain orthopaedic surgery contexts, it is desirable to utilize preoperative and intraoperative images and models of targeted tissue structures, along with instruments registered to coordinate systems common to the instrumentation and anatomy, to predictably address the various targeted tissue structures. Referring to Figure 1, certain aspects of the skeletal anatomy of the shoulder are depicted, including the humerus (2), scapula (4), and collar bone (6). In a scenario wherein an orthopaedic surgery instrument is to be utilized to modify the geometry of one or more portions of this anatomy to repair an injury, prepare for a prosthesis, or other surgical goal, a local mechanical tracker may be utilized intraoperatively to understand the position and orientation of a surgical instrument coupled thereto relative to the skeletal anatomy as the intervention is conducted.

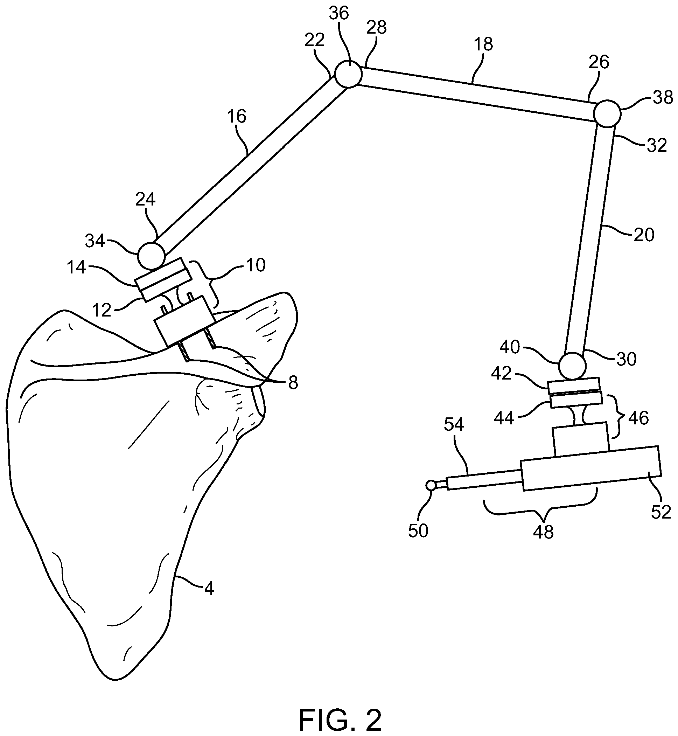

Referring to Figure 2, a configuration is illustrated according to an embodiment wherein a surgical instrument (48), comprising a handle portion (52), an elongate portion (54), and an end effector (50) such as an electromechanically-actuated burr configured to be rotated to remove calcified tissue, is operatively coupled to a portion of the scapula (4) by a mechanical tracker configuration that is local to the operating theater (i.e., it is directly coupled between the surgical instrument 48 and the anatomy directly around the subject of intervention, here the shoulder joint). In the depicted embodiment, the surgical instrument (48) is coupled to the mechanical tracker by an instrument fastener comprising a kinematic interface member (44) that is removably attached to a similar kinematic interface member (42) comprising the distal end of the mechanical tracker. This distal kinematic interface member (42) preferably is coupled, via a rotatable joint (40), to an elongate member (20) that has proximal (32) and distal (30) ends. The proximal end (32) in the depicted embodiment is coupled via another joint (38) to another elongate member (18) having proximal (28) and distal (26) ends, the proximal end (28) of which is coupled, via another joint (36), to a third elongate member (16) which has proximal (24) and distal (22) ends. The proximal end (24) of the most proximal elongate member (16) is coupled to a kinematic interface member (14) by another rotatable joint (34). A skeletal fastener (10) is coupled between the scapula (4) and the proximal end of the mechanical tracker. One or more pins (8) are utilized to fasten the skeletal fastener (10) to the calcified tissue of the scapula (4), while a kinematic interface member (12) is interfaced with the similar kinematic interface member (14) of the proximal end of the mechanical tracker linkage. With adequate degrees of freedom and ranges of motion at each rotatable joint (34, 36, 38, 40) and an understanding of the rotational activity at each such joint, it is possible to have a real-time or near-real-time understanding of the three dimensional spatial positioning and rotation of the instrument relative to the subject anatomy, and this understanding may be utilized, for example, to follow a specific surgical plan. For example, it may be desirable to only remove a specific portion or volume of bone from the subject skeletal anatomy, in accordance with a preoperative or intraoperative plan. While the depicted embodiment shows three elongate members (16, 18, 20) that are rotatably coupled to each other and to fastening configurations (10, 46), other embodiments may contain more or less elongate members and/or joints. Preferably the elongate members are light in weight for relatively low inertial overhead during movement of the surgical tool (48), and are substantially rigid, so that certain assumptions about their deflection during use of the mechanical linkage may be utilized (in another embodiment, they may be more flexible if the flexibility can be characterized with strain gauges or the like, so that deflection of the linkage may be incorporated into the determination of positions and orientations of portions of the linkage).

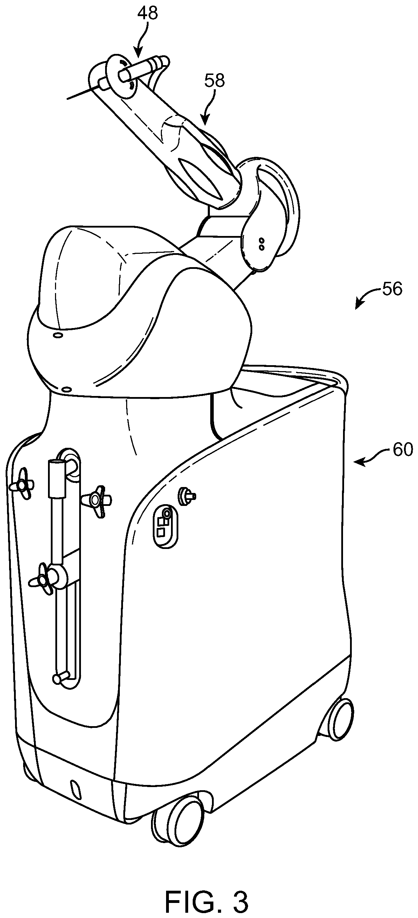

Referring to Figure 3, such a mechanical linkage configuration may be utilized in corporation with a robotic surgical system (56) such as that described, for example, in U.S. Patent 8,010,180, available under the tradename RIO (RTM) from MAKO Surgical Corporation of Ft. Lauderdale, Florida. The depicted instrument (48) may be coupled to the mechanical linkage (and thereby the shoulder) of the configuration of Figure 2, while such instrument (48) also remains coupled to a base controller subsystem (60), which comprises a computerized controller such as a processor or microcontroller, by an instrument support structure (58) such as the depicted robotic arm. The robotic arm may comprise one or more servo motors controlled by the computerized controller, and these servo motors may be selectively activated by the controller to enforce motion limitations upon the surgical instrument (48), such as by providing haptic feedback to an operator whose hand is trying to move the surgical instrument (48), or by providing corrective motion as an operator tries to move the surgical instrument (48) along a path that strays from a predetermined cutting / no-cutting or touch / no-touch plan relative to the pertinent tissue structures. Preferably the kinematics of the instrument support structure and the mechanical tracker linkage may be configured to not spatially intersect or collide with each other for the useful ranges of motion of the surgical instrument (48) in the operating workspace. Such a mechanical linkage configuration also may be utilized in corporation with a freehand surgical tool (i.e., not supported by an instrument support structure) such as those available from Blue Belt Technologies, Inc.

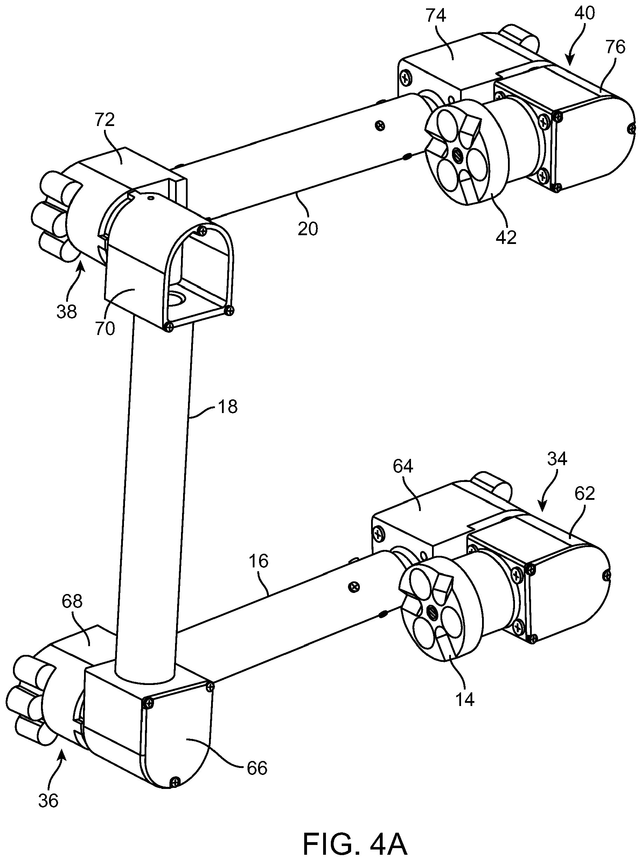

Referring to Figures 4A-4F, various aspects of one embodiment of a mechanical tracker linkage suitable for an interventional configuration such as that depicted in Figure 2 are shown. As shown in Figure 4A, this mechanical tracker embodiment comprises two kinematic interface members (14, 42) rotatably coupled to each other by three elongate members (16, 18, 20) and four joint complexes (34, 36, 38, 40). In further detail, the lower depicted kinematic interface member (14) is rotatably coupled to a housing (62) that is rotatably coupled to another housing (64). This second housing (64) is rotatably coupled to an elongate member (16) which, in this embodiment, is fixedly attached to another housing (68). A rotational degree of freedom arises by the rotational interface between the housing (68) and the housing (66) which is fixedly coupled in this embodiment to the second elongate member (18). The remaining aspects of the depicted mechanical tracker embodiment are fairly homogeneous to those on the other end of the linkage, starting with a housing (70) fixedly coupled to the second elongate member (18), and another rotational joint between the housing (70) and the housing (72). Housing (72) is fixedly coupled to the third elongate member (20), and the other end of the third elongate member (20) is rotatably coupled to another housing (74) which is rotatably coupled to the final housing (76), which is ultimately rotatably coupled to the kinematic interface member (42). The housings (62, 64, 66, 68, 70, 72, 74, 76) in this embodiment are configured to house rotational joint interfaces and associated joint rotation sensors configured to monitor the rotation of each rotational degree of freedom with a high level of precision.

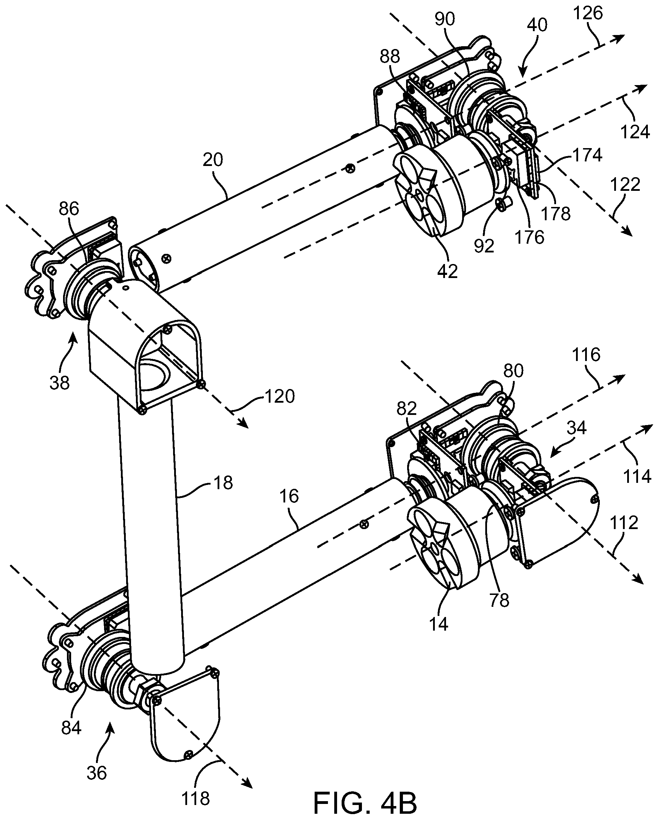

Referring to Figure 4B, a partial cutaway view of an embodiment such as that illustrated in Figure 4A is depicted to show the joint rotation sensors housed in the housings. In this embodiment, the joint rotation sensors are precision digital encoders, such as those available from Hewlett Packard Corporation of Palo Alto, California. As shown in Figure 4B, the first joint complex (34) defines three rotational axes (114, 112, 116), each of which may be monitored by an encoder (78, 80, 82 - respectively). The second joint complex (36) defines a single rotational axis (118) that may be monitored by an encoder (84). The third joint complex (38) defines a single rotational axis (120) that may be monitored by an encoder (86). The fourth joint complex (40) defines three rotational axes (126, 122, 124) that may be monitored by three encoders (88, 90, 92 - respectively). As pointed out in further detail on the third encoder (92) of the fourth joint complex (40), each encoder preferably comprises or is operatively coupled to an encoder board (178), which is coupled to a microcontroller (176) and memory device (174) configured to assist with operation of the encoder (92) and enable it to be operatively coupled, for example, via a wire lead or wireless communication link, to a computerized controller that may reside, for example, in a laptop or desktop computer system, or a computing basestation such as that depicted in Figure 3 (60).

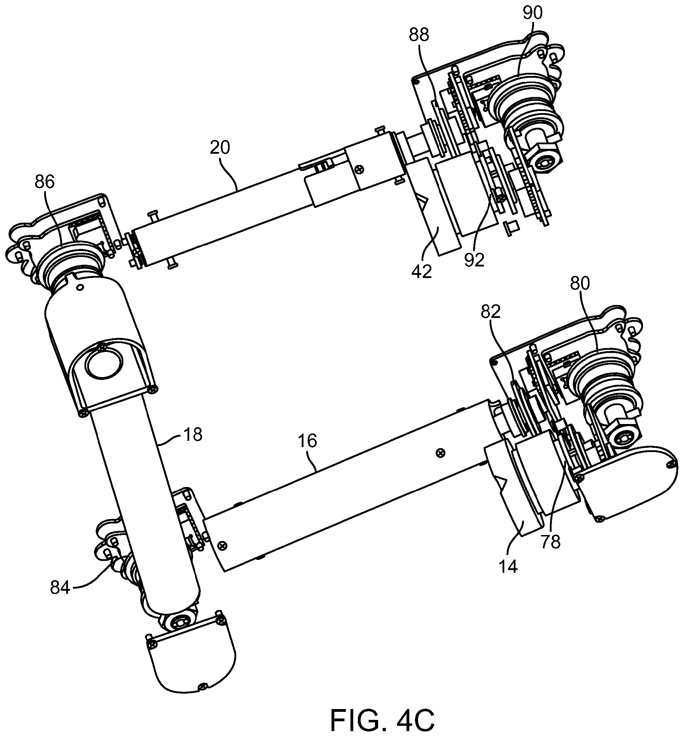

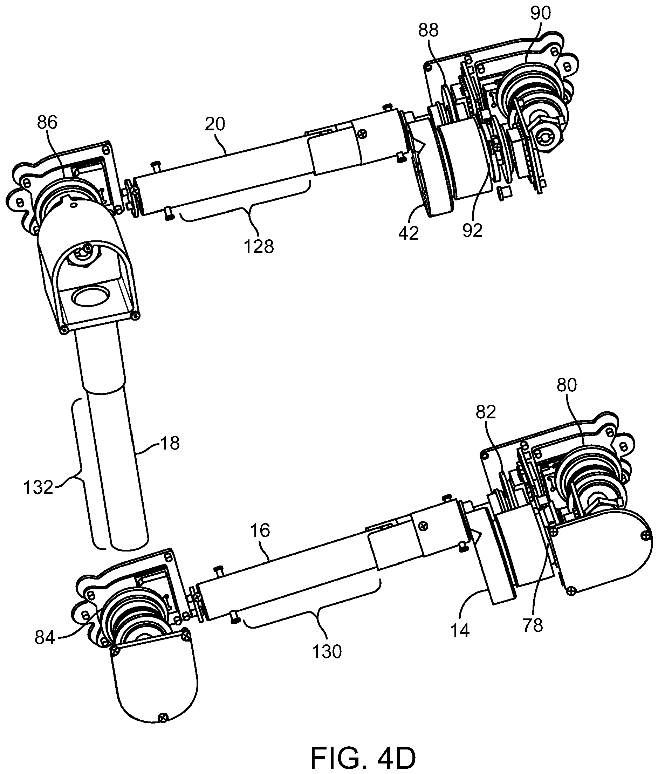

Referring to Figure 4C, a further cutaway view is depicted illustrating the placement of the encoders (78, 80, 82, 84, 86, 88, 90, 92). Referring to Figure 4D, an even further cutaway view of the same embodiment is depicted, showing that one or more batteries (128, 130, 132) may be housed within, or may comprise, one or more of the elongate members (16, 18, 20), to provide the mechanical tracker linkage with a mobile power supply to operate the encoders or other components.

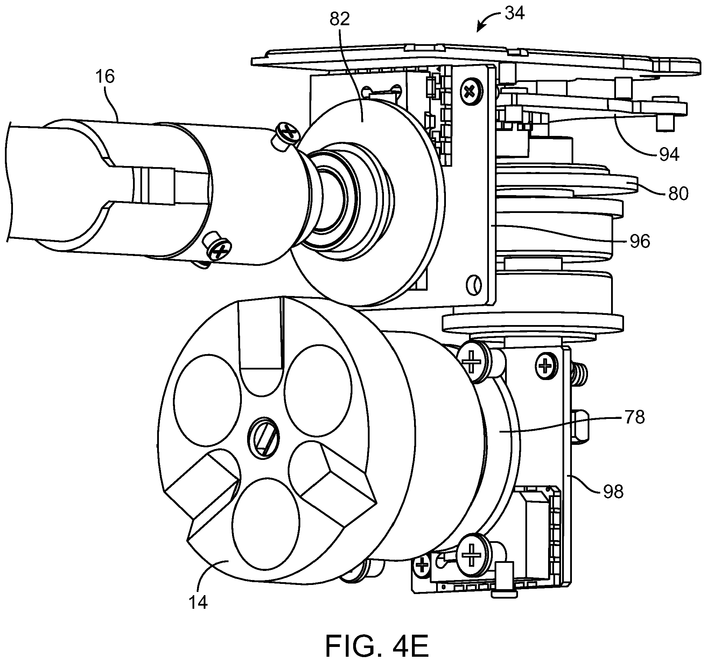

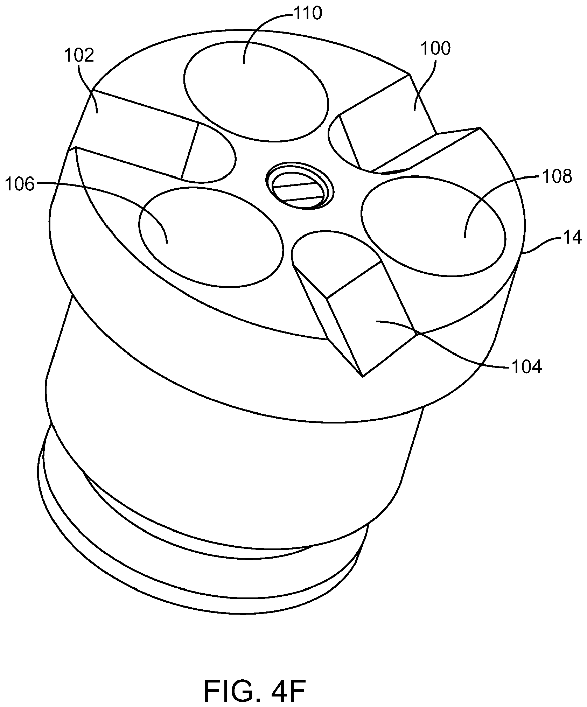

Referring to Figure 4E, a close-up partial cutaway view of the first joint complex (34) is depicted, showing the encoders (78, 80, 82), along with associated encoder boards (98, 94, 96 - respectively). The kinematic interface member (14) is also shown fairly close up, and to illustrate various features of this interface, it is illustrated on its own in Figure 4F. Referring to Figure 4F, this embodiment of a kinematic interface member (14) comprises three ferromagnets (106, 108, 110) and three geometric "female side" grooves (100, 102, 104) that are configured to be removably coupled in a "quick connect" form to a "male side" version featuring similar but opposite polarity magnets in a similar configuration, and protruding elements configured to fit closely into the "female side" grooves (100, 102, 104). The polarities of the magnetic elements preferably are selected to allow only one coupling orientation, so that when the interface (i.e., "male" side and "female" side are coupled, their relative orientation is known). Such a kinematic quick connect interface is configured to reliably provide a tightly toleranced and reliably oriented interface that is easily detached by overpowering the magnetic interfacial loads with an applied load (for example, applied by a surgeon's hand). In one embodiment, substantially all of the main structural parts of the mechanical tracker (with the exception of parts such as the ferromagnetic portions of the quick connect kinematic interface members, certain conductive elements such as wire leads, and certain portions of power supply/battery and rotation sensor components) comprise polymeric materials that may be manufactured in large numbers relatively inexpensively, and that may facilitate a "disposable" mechanical tracker embodiment that may be sterilized and packaged for one use in surgery. Suitable polymers include but are not limited to nylon, glass filled nylon, polyethylene, polystyrene, polyethylene terepthalate, and copolymers thereof.

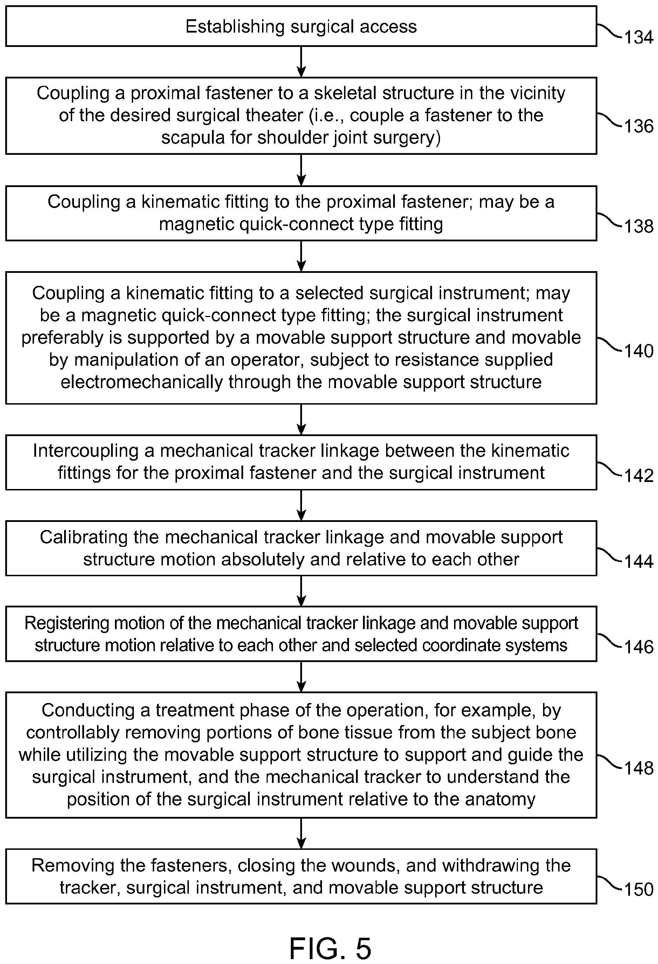

Referring to Figure 5, a process for utilizing a mechanical tracker in a surgical intervention is illustrated. After one or more surgical access points are established (134), for example, by creating one or more incisions or portals, a proximal fastener may be coupled to an exposed skeletal structure (i.e., exposed by the surgical access) in the vicinity of the surgical theater pertinent to the tissue to be surgically altered (136). For example, in a shoulder surgery example, one incision may be created to access a portion of the scapula to fasten a skeletal fastener (for example, using pins 8, as with the fastener 10 configuration depicted in Figure 2), and another incision may be created nearby for access to a bone to be altered with a surgical tool. A kinematic fitting, such as a magnetic quick-connect type kinematic fitting, may be coupled to the skeletal fastener (138). Further, a kinematic fitting, such as a magnetic quick-connect type kinematic fitting, may be coupled to an instrument fastener, which may be coupled to a surgical instrument (140). The surgical instrument is supported by a robotic arm, such as that depicted in Figure 3, wherein robotic arm is movable by manipulation command loads from an operator, subject to resistance that may be provided electromechanically through the robotic arm to the operator as "haptic resistance". A mechanical tracker linkage may be intercoupled between the kinematic fittings coupled to the surgical instrument and skeletal anatomy; in one embodiment, kinematic quick connect interfaces may be selected at the proximal and distal ends of the mechanical tracker to provide for efficient and predictable coupling (142). With the mechanical tracker in place, the surgical instrument may be moved and reoriented in space, for example, in the anticipated surgical workspace for the intervention, while it remains coupled to both the mechanical tracker (which is coupled to the skeletal anatomy) and the movable support structure. A computerized controller operatively coupled to both the mechanical tracker joint sensors and spatial/kinematic information pertinent to the movement of the robotic arm (for example, such as sensing information from an optical tracking system or joint rotation sensor information from an instrument support structure that is to be used in calibration/registration, but that may or may not be utilized in the surgical procedure once the mechanical tracker has been registered/calibrated) may be utilized to observe both data streams and to calibrate or register the two motion tracking systems relative to each other (144, 146).

With the mechanical tracker calibrated and registered, the treatment phase of the surgical operation may be conducted (148) while the joint sensors of the mechanical tracker are utilized to understand the position and orientation of the surgical instrument relative to the anatomy. If an end effector with different geometry is to be utilized, or if a different surgical instrument is to be utilized, calibration/registration may be repeated. In an embodiment wherein kinematic quick connect interfaces are utilized, the proximal end, distal end, or the entire mechanical tracker linkage may be transiently removed from its coupling with the skeletal anatomy or surgical instrument, for example, to accommodate tool switching or inspection with one or more imaging systems that require close in access without a tracker in the immediate workspace. After the surgery has been substantially completed, the systems may be removed with withdrawn from the wound, which may be closed (150).

To further illustrate examples of processes utilizing the subject mechanical tracker technology, descriptions of two exemplary configurations follow. In a first configuration, a robotic surgery system such as that depicted in Figure 3 is to be utilized in an orthopaedic joint intervention - say on the shoulder or the knee. The system comprises a base that may be transiently immobilized relative to a global coordinate system (i.e., such as the floor of an operating room), a controller, and a surgical instrument, such as an electromechanically-actuated rotary burr instrument, that is operatively coupled to the base by a robotic arm comprising a plurality of rigid linkages coupled by joints that are associated with joint rotation sensors. With the base transiently immobilized and the geometry of the instrument relative to the distal end of the robotic arm known, the controller is capable of understanding the position and orientation of the end effector tip of the instrument relative to the global coordinate system associated with the immobilized base. However, this global coordinate system is not necessarily in sync, or in registration, with the coordinate system (or systems) of the tissues to be involved in the surgery - and these coordinate systems are likely to move during surgery as the patient is slightly, or significantly, moved around relative to the floor the operating room, for example. To be able to register and calibrate, and maintain this registration and calibration, of the end effector to a tissue structure, a mechanical tracker may be intercoupled between the tissue structure and the instrument, and joint rotation information from the mechanical tracker may be transmitted (for example, by wire lead or wireless connectivity) to the controller to enable the controller to maintain registration of the instrument and instrument end effector relative to the tissue. Given this registration, the surgical procedure may be undertaken, in accordance, for example, with a predetermined tissue removal plan. If the patient's anatomy moves relative to the global coordinate system, the data from the mechanical tracker is utilized to maintain registration of the end effector relative to the tissue, and in one embodiment, to show an operator intraoperatively where the tool is relative to the anatomy via an image-based three-dimensional virtual environment in a graphical user interface.

In a second exemplary configuration, a handheld ("freehand") surgical instrument is to be utilized without an associated instrument support structure. A mechanical tracker intercoupled between the freehand instrument and the anatomy may be utilized to first establish a calibration / registration with the anatomy, for example, by touching the end effector to known anatomical landmarks, or markers which may have been fastened to the anatomy, of known location. After the instrument is registered to the anatomy, the intervention may be conducted with a controller operatively coupled to the joint sensor information from the mechanical tracker, and this information may be utilized to assist the operator in moving the freehand tool in accordance with a predetermined tissue removal plan, and with the assistance of an image-based three dimensional virtual environment in a graphical user interface, for example.

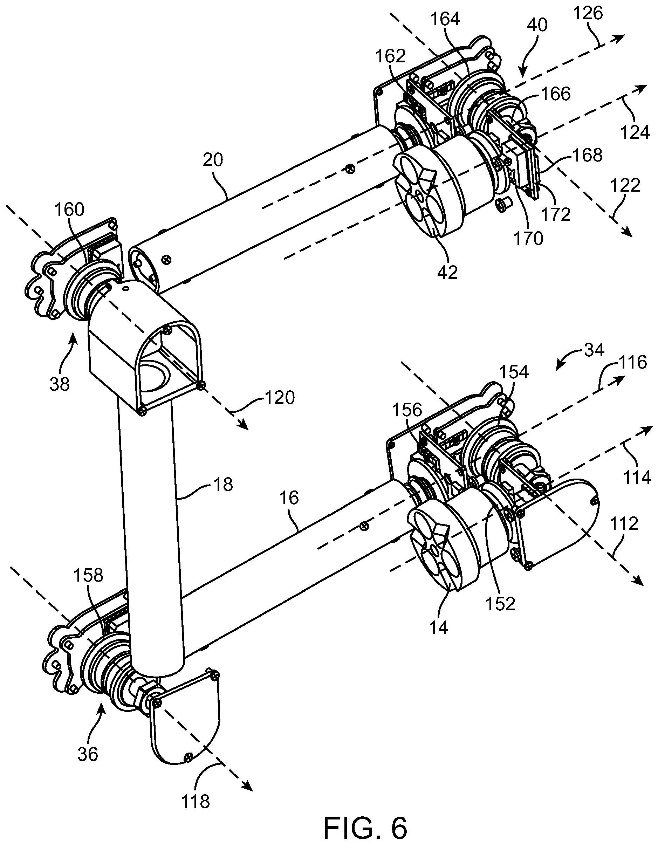

Referring to Figure 6, an embodiment similar to that of Figure 4B is depicted, with the exception that the joint sensors are relatively inexpensive potentiometers (152, 154, 156, 158, 160, 162, 164, 166) rather than encoders as in the embodiment of Figure 4B. Also pointed out in the embodiment of Figure 6 is a board (172), microcontroller (170), and memory device (168) which may be configured particularly for the associated potentiometer (166). In certain configurations, relatively inexpensive potentiometers may be selected as opposed to encoders to decrease the cost of parts and increase the disposability of the mechanical tracker assembly. One of the challenges with potentiometers, however, is that they often have nonlinearities (i.e., voltage emitted from them is not necessarily linearly related with angular deflection). To make the potentiometers more suitable as high-precision joint rotation sensors, as in the subject mechanical tracker configurations, each potentiometer may be characterized relative to a high precision joint rotation sensor such as a precision encoder, and the nonlinearities may be mapped to equations, characterized in lookup tables, etc., so that the voltages may be accurately mapped to joint rotation angles and thereby be utilized by an associated controller to assist in the understanding of position and orientation of an associated surgical instrument relative to the pertinent anatomical structures.

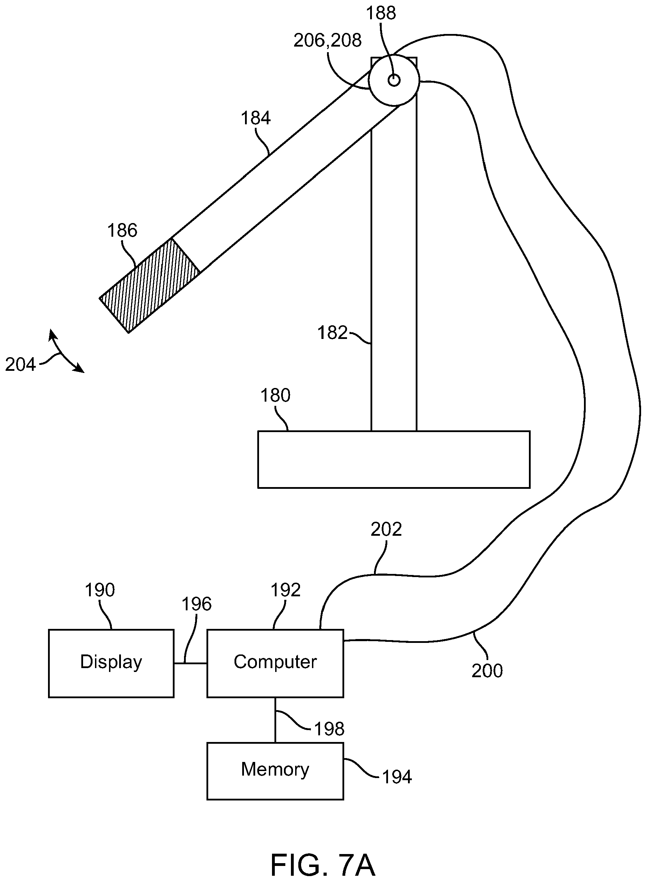

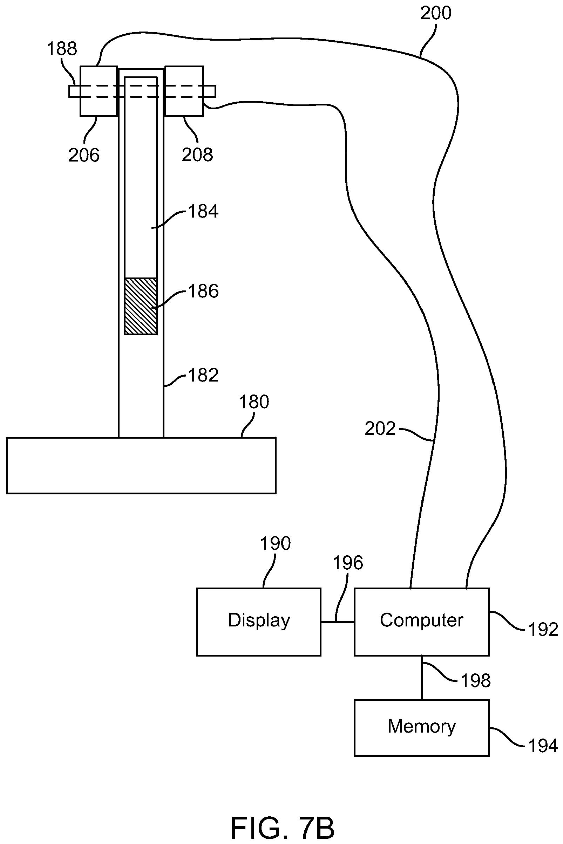

Referring to Figures 7A and 7B, two orthogonal views of a configuration for characterizing a potentiometer using a precision encoder are depicted. The depicted apparatus comprises a base member (180) and stand member (182) coupled to a rotatable member (184) with a rotational joint about an axle (188) that causes both an associated precision encoder (206) and a potentiometer (208) to rotate together. The rotational member may be manually rotated (204) by manipulation of the handle (186). Both the encoder (206) and the potentiometer (208) are coupled via wire leads (202, 200) to a computing system (192) comprising a controller or microcontroller, that preferably is coupled via wire leads (196, 198) to a display (190) and memory device (194). As the rotational member (184) is rotated (204), the voltage output of the potentiometer (208) may be compared and characterized relative to the output and associated angular rotation position of the encoder (206), and mathematical techniques, such as polynomial fitting, may be utilized to develop predictable mathematical relationships, or lookup tables, that may be utilized subsequently to determine rotational angle positions from the potentiometer (208) without further assistance of the encoder (206). The information (for example, mathematical relationships or lookup tables) characterizing each potentiometer may be stored on a memory local to the board and microcontroller associated with each potentiometer incorporated into a mechanical tracker assembly.

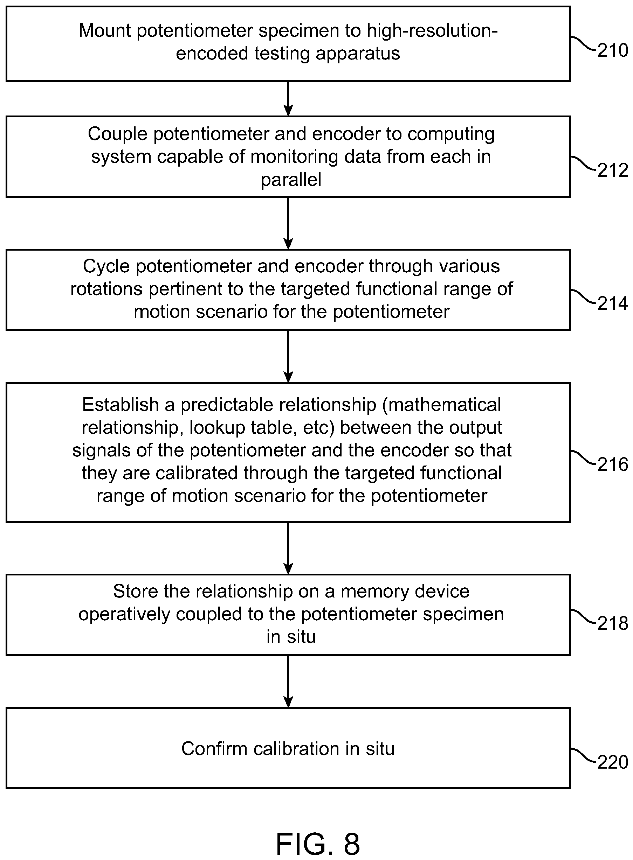

Referring to Figure 8, a process as described above for calibrating and characterizing a potentiometer for use as an accurate joint rotation sensor is illustrated. A potentiometer specimen is coupled to a high-resolution-encoded testing apparatus (210). Both the potentiometer and encoder are coupled to a computing system capable of monitoring signals from both in parallel (212). The potentiometer and encoder and rotationally cycled through a given range of motion scenario (preferably at least close to the targeted range of motion scenario for functionality of the mechanical tracker) (214), and a relationship is established to be able to associate potentiometer output voltages with joint rotation angle in the targeted range of motion scenario (216). The characterization information (such as polynomial equations fitted to the output voltages and encoder angles, lookup tables, etc.) preferably is stored on a memory device operatively coupled to the potentiometer specimen (for example, on an associated board, or on an associated memory device operatively coupled to the controller or computing system) (218). Calibration may be confirmed in situ as well (220), by checking known joint rotations with rotation feedback determined using the potentiometer characterization information.

Various exemplary embodiments of the invention are described herein. Reference is made to these examples in a non-limiting sense. They are provided to illustrate more broadly applicable aspects of the invention. The scope of the invention is defined by the appended claims. Various changes may be made to the invention described within the scope of claims associated with this disclosure.

Any of the devices described for carrying out the subject interventions may be provided in packaged combination for use in executing such interventions. These supply "kits" further may include instructions for use and be packaged in sterile trays or containers as commonly employed for such purposes.

Methods recited herein may be carried out in any order of the recited events which is logically possible, as well as in the recited order of events.

Exemplary aspects of the invention, together with details regarding material selection and manufacture have been set forth above. As for other details of the present invention, these may be appreciated as generally known or appreciated by those with skill in the art. For example, one with skill in the art will appreciate that one or more lubricious coatings (e.g., hydrophilic polymers such as polyvinylpyrrolidone-based compositions, fluoropolymers such as tetrafluoroethylene, hydrophilic gel or silicones) or polymer parts suitable for use as low friction bearing surfaces (such as ultra high molecular weight polyethylene) may be used in connection with various portions of the devices, such as relatively large interfacial surfaces of movably coupled parts, if desired, for example, to facilitate low friction manipulation or advancement of such objects relative to other portions of the instrumentation or nearby tissue structures. The same may hold true with respect to method-based examples in terms of additional acts as commonly or logically employed.

Where a range of values is provided, it is understood that every intervening value, between the upper and lower limit of that range and any other stated or intervening value in that stated range, is encompassed within the invention. Reference to a singular item, includes the possibility that there are plural of the same items present. More specifically, as used herein and in claims associated hereto, the singular forms "a," "an," "said," and "the" include plural referents unless specifically stated otherwise. In other words, use of the articles allow for "at least one" of the subject item in the description above as well as claims associated with this disclosure.

## Claims

1. A robotic surgery system, comprising:
a controller (192) configured to control actuation of at least one servo motor; and
a surgical instrument (48) coupled distally to a mechanical tracker linkage (16, 18, 20, 34, 36, 38, 40) and proximally to a robotic arm (58) coupled to the at least one servo motor, such that the surgical instrument (48) is configured to be movable in a workspace controlled, at least in part, by actuation of the at least one servo motor;
wherein the mechanical tracker linkage (16, 18, 20, 34, 36, 38, 40) is adapted to be coupled between the surgical instrument (48) and a portion of skeletal anatomy of a patient, the tracker linkage comprising one or more joints associated with one or more joint rotation sensors and being configured to send joint signals to the controller;
wherein the controller controls the positioning of the instrument (48) by selectively activating the at least one servo motor coupled to the robotic arm (58) to enforce motion limitations upon the surgical instrument (48) based at least in part upon the joint signals received from the mechanical tracker linkage (16, 18, 20, 34, 36, 38, 40).

2. The system of claim 1, wherein the surgical instrument comprises a bone removal instrument, optionally a electromechanically-actuated burr.

3. The system of claim 1, wherein the surgical instrument is coupled to an immobilized base unit (60) by the robotic arm.

4. The system of claim 1, wherein the controller is configured to provide haptic feedback to an operator handling the surgical instrument by controlled actuation of the one or more servo motors.

5. The system of claim 1, wherein the controller is configured to provide corrective motion to the surgical instrument by controlled actuation of the one or more servo motors.

6. The system of claim 1, wherein the mechanical tracker linkage comprises at least two substantially rigid portions (16, 18) coupled by at least one movable joint (36).

7. The system of claim 6, wherein the mechanical tracker linkage comprises at least three substantially rigid portions (16, 18, 20) coupled in a series configuration by two or more movable joints (34, 36, 38, 40).

8. The system of claim 7, wherein the series configuration comprises a proximal end and a distal end, each of which is coupled to a kinematic quick-connect fitting (14, 42).

9. The system of claim 8, wherein:-
(i) a proximal kinematic quick-connect fitting is configured to be fixedly and removably coupled to a skeletal bone; or
(ii) a distal kinematic quick-connect fitting is configured to be fixedly and removably coupled to the surgical instrument.

10. The system of claim 9, wherein the proximal kinematic quick-connect fitting is configured to be fixedly and removably coupled to the skeletal bone using an additional kinematic quick-connect fitting coupled to the skeletal bone.

11. The system of claim 9, wherein the distal kinematic quick-connect fitting is configured to be fixedly and removably coupled to the surgical instrument using an additional kinematic quick-connect fitting coupled to the surgical instrument.

12. The system of claim 10, wherein the proximal and additional kinematic quick-connect fittings are biased to stay in a coupled configuration by one or more magnets (106, 108, 110) associated with one or more kinematic orienting surfaces.

13. The system of claim 11, wherein the distal and additional kinematic quick-connect fittings are biased to stay in a coupled configuration by one or more magnets associated with one or more kinematic orienting surfaces.

14. The system of claim 10, wherein the additional kinematic quick-connect fitting is coupled to one or more pins, which are fastened directly to the skeletal bone.

15. The system of claim 1, wherein:-
(i) at least one of the one or more joint rotation sensors comprises an encoder (206); or
(ii) at least one of the one or more joint rotation sensors comprises a potentiometer (208).

16. The system of claim 1, wherein the mechanical tracker linkage comprises an on-board power supply configured to power the one or more joint rotation sensors.

17. The system of claim 1, wherein the tracker linkage comprises a disposable polymeric material selected from the group consisting of: nylon, glass filled nylon, polyethylene terepthalate, polystyrene, polyethylene, and copolymers thereof.

## Patentansprüche

1. Roboterchirurgie-System, umfassend:
eine Steuerung (192), die zur Steuerung der Betätigung mindestens eines Servomotors ausgelegt ist; und
ein chirurgisches Instrument (48), das distal mit einem mechanischen Tracker-Gestänge (16, 18, 20, 34, 36, 38, 40) und proximal mit einem, mit dem mindestens einen Servomotor verbundenen Roboterarm (58) verbunden ist, so dass das chirurgische Instrument (48) dazu ausgelegt ist, in einem Arbeitsraum bewegbar zu sein, zumindest teilweise gesteuert durch Betätigung des mindestens einen Servomotors;
wobei das mechanische Tracker-Gestänge (16, 18, 20, 34, 36, 38, 40) zur Kupplung zwischen dem chirurgischen Instrument (48) und einem Teil der Skelettanatomie eines Patienten ausgelegt ist, wobei das Tracker-Gestänge ein oder mehrere Gelenke umfasst, die mit einem oder mehreren Gelenkdrehsensoren in Verbindung stehen und dazu ausgelegt sind, Gelenksignale an die Steuerung zu senden;
wobei die Steuerung die Positionierung des Instruments (48) durch selektive Aktivierung des mindestens einen, mit dem Roboterarm (58) verbundenen Servomotors steuert, um dem chirurgischen Instrument (48) Bewegungseinschränkungen aufzuzwingen, zumindest teilweise basierend auf den vom mechanischen Tracker-Gestänge (16, 18, 20, 34, 36, 38, 40) empfangenen Gelenksignalen.

2. System nach Anspruch 1, wobei das chirurgische Instrument ein Knochenentfernungsinstrument umfasst, optional eine elektromechanisch betätigte Fräse.

3. System nach Anspruch 1, wobei das chirurgische Instrument über den Roboterarm mit einer immobilisierten Basiseinheit (60) verbunden ist.

4. System nach Anspruch 1, wobei die Steuerung zur Bereitstellung einer haptischen Rückmeldung an eine das chirurgische Instrument handhabende Bedienungsperson durch kontrollierte Betätigung des einen oder der mehreren Servomotoren ausgelegt ist.

5. System nach Anspruch 1, wobei die Steuerung zur Bereitstellung einer korrigierenden Bewegung an das chirurgische Instrument durch kontrollierte Betätigung des einen oder der mehreren Servomotoren ausgelegt ist.

6. System nach Anspruch 1, wobei das mechanische Tracker-Gestänge mindestens zwei im Wesentlichen starre Abschnitte (16, 18) umfasst, die über mindestens ein bewegliches Gelenk (36) miteinander verbunden sind.

7. System nach Anspruch 1, wobei das mechanische Tracker-Gestänge mindestens drei im Wesentlichen starre Abschnitte (16, 18, 20) umfasst, die in einer Reihenkonfiguration über zwei oder mehr bewegliche Gelenke (34, 36, 38, 40) miteinander verbunden sind.

8. System nach Anspruch 7, wobei die Reihenkonfiguration ein proximales Ende und ein distales Ende umfasst, die jeweils mit einer kinematischen Schnellkupplung (14, 42) verbunden sind.

9. System nach Anspruch 8, wobei:-
(i) eine proximale kinematische Schnellkupplung zur fixierten und entfernbaren Verbindung mit einem Skelettknochen ausgelegt ist; oder
(ii) eine distale kinematische Schnellkupplung zur fixierten und entfernbaren Verbindung mit dem chirurgischen Instrument ausgelegt ist.

10. System nach Anspruch 9, wobei die proximale kinematische Schnellkupplung zur fixierten und entfernbaren Verbindung mit dem Skelettknochen unter Verwendung einer zusätzlichen kinematischen Schnellkupplung, die mit dem Skelettknochen verbunden ist, ausgelegt ist.

11. System nach Anspruch 9, wobei die distale kinematische Schnellkupplung zur fixierten und entfernbaren Verbindung mit dem chirurgischen Instrument unter Verwendung einer zusätzlichen kinematischen Schnellkupplung, die mit dem chirurgischen Instrument verbunden ist, ausgelegt ist.

12. System nach Anspruch 10, wobei die proximalen und zusätzlichen kinematischen Schnellkupplungen von einem oder mehreren Magneten (106, 108, 110), die mit einer oder mehreren kinematischen Orientierungsflächen in Zusammenhang stehen, so vorgespannt werden, dass sie in einer verbundenen Konfiguration bleiben.

13. System nach Anspruch 11, wobei die distalen und zusätzlichen kinematischen Schnellkupplungen von einem oder mehreren Magneten, die mit einer oder mehreren kinematischen Orientierungsflächen in Zusammenhang stehen, so vorgespannt werden, dass sie in einer verbundenen Konfiguration bleiben.

14. System nach Anspruch 10, wobei die zusätzliche kinematischen Schnellkupplung mit einem oder mehreren Stiften, die direkt am Skelettknochen befestigt sind, verbunden sind.

15. System nach Anspruch 1, wobei:-
(i) mindestens einer der ein oder mehreren Gelenkdrehungssensoren einen Codierer (206) umfasst; oder
(ii) mindestens einer der ein oder mehreren Gelenkdrehungssensoren einen Potentiometer (208) umfasst.

16. System nach Anspruch 1, wobei das mechanische Tracker-Gestänge eine Onboard-Stromversorgung umfasst, die zum Antreiben der ein oder mehreren Gelenkdrehungssensoren ausgelegt ist.

17. System nach Anspruch 1, wobei das Tracker-Gestänge ein Einweg-Polymermaterial umfasst, das aus der Gruppe ausgewählt ist, die besteht aus: Nylon, glasgefülltes Nylon, Polyethylenterephthalat, Polystyrol, Polyethylen und Copolymere davon.

## Revendications

1. Système de chirurgie robotique, comprenant :
un contrôleur (192) configuré pour commander l'actionnement d'au moins un servomoteur; et
un instrument chirurgical (48) couplé distalement à une liaison de dispositif de suivi mécanique (16, 18, 20, 34, 36, 38, 40) et proximalement à un bras robotique (58) couplé audit au moins un servomoteur, de telle sorte que l'instrument chirurgical (48) est configuré pour être mobile dans un espace de travail commandé, au moins en partie, par l'actionnement dudit au moins un servomoteur;
dans lequel la liaison de dispositif de suivi mécanique (16, 18, 20, 34, 36, 38, 40) est adaptée pour être couplée entre l'instrument chirurgical (48) et une partie de l'anatomie squelettique d'un patient, la liaison de dispositif de suivi comprenant une ou plusieurs articulations associées à un ou plusieurs capteurs de rotation d'articulation et étant configurée pour envoyer des signaux d'articulation au contrôleur;
dans lequel le contrôleur commande le positionnement de l'instrument (48) en activant sélectivement ledit au moins un servomoteur couplé au bras robotique (58) pour imposer des limitations de mouvement sur l'instrument chirurgical (48) sur la base, au moins en partie, des signaux d'articulation reçus de la liaison de dispositif de suivi mécanique (16, 18, 20, 34, 36, 38, 40).

2. Système selon la revendication 1, dans lequel l'instrument chirurgical comprend un instrument d'ablation d'os, éventuellement une fraise actionnée électromécaniquement.

3. Système selon la revendication 1, dans lequel l'instrument chirurgical est couplé à une unité de base immobilisée (60) par le bras robotique.

4. Système selon la revendication 1, dans lequel le contrôleur est configuré pour fournir une rétroaction haptique à un opérateur manipulant l'instrument chirurgical par actionnement contrôlé d'un ou plusieurs servomoteurs.

5. Système selon la revendication 1, dans lequel le contrôleur est configuré pour fournir un mouvement correctif à l'instrument chirurgical par actionnement contrôlé d'un ou plusieurs servomoteurs.

6. Système selon la revendication 1, dans lequel la liaison de dispositif de suivi mécanique comprend au moins deux parties sensiblement rigides (16, 18) couplées par au moins un joint mobile (36).

7. Système selon la revendication 6, dans lequel la liaison de dispositif de suivi mécanique comprend au moins trois parties sensiblement rigides (16, 18, 20) couplées en série par au moins deux joints mobiles (34, 36, 38, 40).

8. Système selon la revendication 7, dans lequel la configuration en série comprend une extrémité proximale et une extrémité distale, dont chacune est couplée à un raccord rapide cinématique (14, 42).

9. Système selon la revendication 8, dans lequel :
(i) un raccord rapide cinématique proximal est configuré pour être couplé de façon fixe et amovible à un os squelettique; ou
(ii) un raccord rapide cinématique distal est configuré pour être couplé de manière fixe et amovible à l'instrument chirurgical.

10. Système selon la revendication 9, dans lequel le raccord rapide cinématique proximal est configuré pour être couplé de manière fixe et amovible à l'os squelettique en utilisant un raccord rapide cinématique supplémentaire couplé à l'os squelettique.

11. Système selon la revendication 9, dans lequel le raccord rapide cinématique distal est configuré pour être couplé de manière fixe et amovible à l'instrument chirurgical à l'aide d'un raccord rapide cinématique supplémentaire couplé à l'instrument chirurgical.

12. Système selon la revendication 10, dans lequel les raccords rapides cinématiques proximaux et supplémentaires sont polarisés pour rester dans une configuration couplée par un ou plusieurs aimants (106, 108, 110) associés à une ou plusieurs surfaces d'orientation cinématique.

13. Système selon la revendication 11, dans lequel les raccords rapides cinématiques distaux et supplémentaires sont polarisés pour rester dans une configuration couplée par un ou plusieurs aimants associés à une ou plusieurs surfaces d'orientation cinématique.

14. Système selon la revendication 10, dans lequel le raccord rapide cinématique supplémentaire est couplé à une ou plusieurs broches qui sont fixées directement sur l'os squelettique.

15. Système selon la revendication 1, dans lequel :
(i) au moins l'un desdits au moins un capteur de rotation d'articulation comprend un codeur (206); ou
(ii) au moins l'un desdits au moins un capteur de rotation d'articulation comprend un potentiomètre (208) .

16. Système selon la revendication 1, dans lequel la liaison de dispositif de suivi mécanique comprend une alimentation électrique embarquée configurée pour alimenter lesdits au moins un capteur de rotation d'articulation.

17. Système selon la revendication 1, dans lequel la liaison du dispositif de suivi comprend un matériau polymère jetable choisi dans le groupe constitué par : le nylon, le nylon chargé de verre, le polyéthylène téréphtalate, le polystyrène, le polyéthylène et leurs copolymères.
